(19) Europäisches Patentamt
European Patent Office
Office européen des brevets

(11) Veröffentlichungsnummer: **0 132 688**
**B1**

(12) # EUROPÄISCHE PATENTSCHRIFT

(45) Veröffentlichungstag der Patentschrift: **23.11.88**

(21) Anmeldenummer: **84108046.8**

(22) Anmeldetag: **10.07.84**

(51) Int. Cl.⁴: **C 07 B 37/04,** C 07 C 49/12, C 07 C 69/34, C 07 C 69/716, C 07 C 121/16, C 07 C 121/20, C 07 C 121/66, C 07 D 239/62, C 07 C 45/68, C 07 C 67/343, C 07 C 120/00

(54) **Verfahren zur Herstellung von monomethylsubstituierten Methylen-Verbindungen.**

(30) Priorität: **23.07.83 DE 3326635**

(43) Veröffentlichungstag der Anmeldung: **13.02.85 Patentblatt 85/7**

(45) Bekanntmachung des Hinweises auf die Patenterteilung: **23.11.88 Patentblatt 88/47**

(84) Benannte Vertragsstaaten: **CH DE FR GB LI**

(56) Entgegenhaltungen:
**EP-A-0 028 703**
**EP-A-0 100 019**
**FR-A-2 073 109**
**FR-A-2 268 793**

(73) Patentinhaber: **BAYER AG, Konzernverwaltung RP Patentabteilung, D-5090 Leverkusen 1 Bayerwerk (DE)**

(72) Erfinder: **Blank, Heinz Ulrich, Dr., Am Geusfelde 35, D-5068 Odenthal (DE)**
Erfinder: **Wolters, Erich, Dr., Streffenweg 22, D-5162 Niederzier (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 132 688 B1

LIBER, STOCKHOLM 1988

**Beschreibung**

Es ist bekannt, C-H-acide Verbindungen, wie Cyanessigester, Malonester oder Acetessigester, mit Methylhalogeniden, wie Methylbromid, oder mit Methylestern, wie Dimethylsulfat, zu den monomethylierten Verbindungen umzusetzen. Ein großer Nachteil dieser Arbeitsweise besteht darin, daß dabei immer Gemische der monomethylierten Verbindungen mit der Ausgangsverbindung und mit dem dimethylierten Produkt erhalten werden, die wegen ihrer sich nur geringfügig unterscheidenden Siedepunkte erst durch großen destillativen Aufwand getrennt werden können. Für die Herstellung reiner monomethylierter Verbindungen ist dieses Verfahren daher nicht besonders gut geeignet.

Zur Alkylierung C-H-acider Verbindungen ist ferner unter dem Namen "Reduktive Alkylierung" die Methode bekannt, diese Verbindungen mit Aldehyden oder Ketonen zu den Alkylidenderivaten zu kondensieren und diese dann durch Hydrierung in die gewünschten Alkylderivate zu überführen (Org. Reactions 9, S. 145, 146). Diese Reaktion wird mit Vorteil einstufig durchgeführt, wobei die Hydrierung der zu Polymerisation neigenden Alkylidenderivate in Gegenwart von Wasserstoff und eines Hydrierkatalysators unmittelbar nach der Kondensation erfolgen kann. Insbesondere bei Verwendung der niederen aliphatischen Aldehyde werden nach diesem Verfahren die Monoalkylderivate in hohen Ausbeuten (vielfach über 90 % der theoretischen Ausbeute) erhalten (J. Am. Chem. Soc. 66, 886-888 (1944); DE-OS-2 060 443). Im Gegensatz zur Verwendung anderer niederer aliphatischer Aldehyde führt jedoch die Methylierung unter Einsatz von Formaldehyd zu deutlich geringeren Ausbeuten. So werden in der DE-OS-2 060 443 bei der Methylierung von Acetessigester nach diesem Verfahren Ausbeuten von nur 40 - 45 % beschrieben. In der DE-OS-2 515 039 werden bei der Methylierung von Cyanessigester nach diesem Verfahren Ausbeuten zwischen 14 und 61 % genannt. In allen beschriebenen Fällen erfolgt die Umsetzung in der Weise, daß die Reaktionsteilnehmer zusammen vorgelegt werden oder daß der Formaldehyd im Verlaufe der Reaktion zum Gemisch der übrigen Reaktionspartner zugefügt wird.

Es wurde nun ein Verfahren zur Herstellung von monomethylsubstituierten Methylen-Verbindungen der Formel

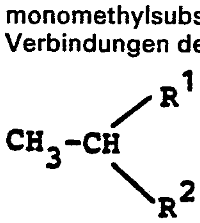

(I)

durch Methylierung von Methylen-Verbindungen der Formel

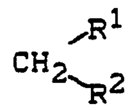

(II)

in der

R¹ und R² unabhängig voneinander für -CN, -CO-$R^3$, -$SO_2$-$R^3$ und -$NO_2$ stehen und $R^1$ zusätzlich Aryl$(R^1)_n$ bedeuten kann, wobei

$R^3$ -OH, Alkyl, Aralkyl, Aryl, Alkoxy, Aralkoxy, Aryloxy oder gegebenenfalls durch Alkyl und/oder Aralkyl und/oder Aryl substituiertes Amino bedeutet und weiterhin zwei Reste $R^3$ gemeinsam eine Alkylengruppe, der Rest eines aliphatischen Diols oder eines aliphatischen Diamins oder die Gruppe -NH-CO-NH sein können und

n für 1, 2 oder 3 steht,

durch Reaktion mit Formaldehyd und Wasserstoff in Gegenwart eines Kondensationskatalysators und eines Hydrierkatalysators bei erhöhter Temperatur gefunden, daß dadurch gekennzeichnet ist, daß die Reaktion in einem Lösungsmittel durchgeführt wird und die Methylen-Verbindung im Verlauf der Reaktion der flüssigen Phase des Gemisches der Reaktionsteilnehmer zugefügt wird oder eine im Lösungsmittel sehr schwer lösliche Methylen-Verbindung gemeinsam mit den anderen Reaktionsteilnehmern vorgelegt wird, die im Verlauf der Reaktion in die flüssige Phase übergeht.

Als Alkyl sei ein geradkettiger oder verzweigter aliphatischer Kohlenwassertoffrest mit 1 - 10, bevorzugt 1 - 4, besonders bevorzugt 1 - 2 Kohlenstoffatomen genannt, wie Methyl, Ethyl, Propyl, Isopropyl, Butyl, Isobutyl, tert.-Butyl, Hexyl, Octyl oder Decyl.

Als Aralkyl sei beispielsweise Benzyl, Phenylethyl oder Phenylpropyl, bevorzugt Benzyl, genannt.

Als Aryl sei beispielsweise Phenyl oder Naphthyl, bevorzugt Phenyl, genannt, wobei der aromatische Kern ebenso wie in den genannten Aralkylresten noch mit $C_1$-$C_4$-Alkyl, $C_1$-$C_4$-Alkoxy, $C_1$-$C_4$-alkyl-amino oder Halogen, wie Fluor, Chlor, Brom oder Jod, bevorzugt Fluor oder Chlor, substituiert sein kann.

Als Alkoxy, Aralkoxy oder Aryloxy seien beispielsweise das um ein Sauerstoff ergänzte vorgenannte Alkyl, Aralkyl oder Aryl genannt, wobei die genannten Vorzugsbereiche für die Anzahl der C-Atome gelten.

Der Index n kann den Zahlenwert 1, 2 oder 3 annehmen, wobei der Zahlenwert 1 bevorzugt ist. Von den durch den Index n gekennzeichneten Substituenten $R^1$ für den Arylrest steht bevorzugt wenigstens einer in o- oder p-Stellung.

Zwei Reste $R^3$ können weiterhin gemeinsam eine Alkylengruppe, beispielsweise mit 2 - 5, bevorzugt 3 - 4 C-Atomen, der Rest eines

aliphatischen Diols oder aliphatischen Diamins, beispielsweise mit jeweils 2 - 4, bevorzugt 2 - 3 C-Atomen, oder die Gruppe -NH-CO-NH- sein. Durch eine solche Konfiguration entstehen cyclische Methylen-Verbindungen, die erfindungsgemäß umgesetzt werden können. Selbstverständlich können die H-Atome an den C- oder N-Atomen solcher aus zwei Resten $R^3$ gebildeter Ringglieder durch inerte Atome oder Gruppen ersetzt sein, wie beispielsweise Fluor, Chlor, Brom, $C_1$-$C_4$-Alkyl, $C_1$-$C_4$-Alkoxy oder $C_1$-$C_4$-Alkyl-amino.

Als erfindungsgemäß einsetzbare Methylen-Verbindungen seien bevorzugt solche der Formel

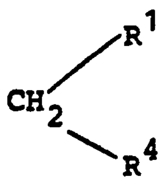

(III)

genannt, in der

$R^1$ die oben genannte Bedeutung hat und

$R^4$ für -CN oder -CO-$R^3$ steht, wobei $R^3$ ebenfalls die oben genannte Bedeutung hat.

In besonders bevorzugter Weise werden Methylen-Verbindungen der Formel

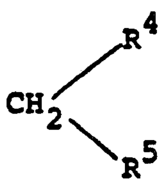

(IV)

eingesetzt, in der

$R^4$ und $R^5$ unabhängig voneinander für -CN oder -CO-$R^3$ stehen, wobei $R^3$ die oben genannte Bedeutung hat.

In ganz besonders bevorzugter Weise werden Methylen-Verbindungen der Formel

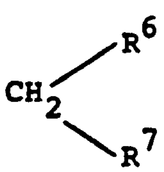

(V)

eingesetzt, in der

$R^6$ und $R^7$ unabhängig voneinander -CN,-CO-$C_1$-$C_4$-Alkyl oder -CO-O-$C_1$-$C_4$-Alkyl bedeuten.

Beispiele für erfindungsgemäß einsetzbare Verbindungen sind: Malodinitril, Cyanessigester mit verschiedenen alkoholischen Resten, Acetessigester mit verschiedenen alkoholischen Resten, Nitroessigsäureester mit verschiedenen alkoholischen Resten, Malonsäureester mit verschiedenen alkoholischen Resten, Malonsäure, Cyanessigsäure, Acetessigsäure, Cyclopentandion(1,3), Cyclohexandion(1,3), cyclischer Malonsäure-ethylenglykolester, cyclisches Malonsäure-ethylendiamid, Barbitursäure, Acetylaceton, Cyanmethansulfonsäureester mit verschiedenen alkoholischen Resten, 4-Nitrobenzylcyanid, 2,4-Dinitrobenzylcyanid und 4-Carbethoxyphenylessigsäureester mit verschiedenen alkoholischen Resten.

Das erfindungsgemäße Verfahren kann bei einem Wasserstoffpartialdurck von 1 - 100 bar oder mehr durchgeführt werden. Bevorzugt wird der Druckbereich von 3 bis 50 bar, besonders bevorzugt von 5 bis 15 bar eingestellt.

Als Hydrierungskatalysatoren eignen sich alle üblichen Edelmetallkatalysatoren, wie Palladium, Platin, Rhodium oder Rhuthenium, bevorzugt auf Trägermaterialien, wie Kieselsäure, Aluminiumoxid oder Kohle. Möglich ist weiterhin die Verwendung von Raney-Nickel oder Raney-Kobalt und verwandten Raney-Katalysatoren. Bevorzugt wird ein Katalysator aus Palladium auf Kohle, besonders bevorzugt mit einem Palladiumgehalt von 3 - 5 Gew.-% des gesamten Trägerkatalysators eingesetzt. Die Menge des Katalysators kann bei 1 - 20 Gew.-%, bezogen auf die eingesetzte Methylen-Verbindung liegen. Vielfach sind 3 - 10 Gew.-%, bevorzugt 4 - 6 Gew.-% Katalysator, bezogen auf eingesetzte Methylen-Verbindung, ausreichend.

Die Reaktionstemperatur liegt bei 30 - 130° C, bevorzugt 60 - 100° C, besonders bevorzugt bei 80 - 90° C.

Als Kondensationskatalysatoren können solche verwendet werden, die Reaktionen vom Typ der Aldolkondensation katalysieren. Solche Katalysatoren sind beispielsweise Amine, wie Piperidin, Morpholin, Pyrrolidin, Triethylamin oder andere tertiäre Amine oder basisch reagierende Salze, beispielsweise von organischen Säuren, wie Natriumacetat, Kaliumoxalat oder Carbonsäuren, wie Ameisensäure, Essigsäure oder Chlorwasserstoff oder Schwefelsäure oder Lewissäuren, wie Zinkchlorid, Aluminiumchlorid oder Eisenchlorid.

Als Kondensationkatalysatoren werden bevorzugt tertiäre Amine, die genannten basisch reagierenden Salze oder Essigsäure eingesetzt. Die Menge des eingesetzten Kondensationskatalysators liegt im allgemeinen bei 1 - 10 Mol-%, bezogen auf die eingesetzte Methylen-Verbindung, bevorzugt bei 3 - 6 Mol-%. Der optimale Kondensationskatalysator und die optimale Katalysatormenge könne im Einzelfall leicht durch Vorversuche ermittelt werden.

Der Formaldehyd kann als Paraformaldehyd, als Formaldehydgas oder als Formaldehydlösung, beispielsweise in Alkoholen, wie Methanol, eingesetzt werden. Der Einsatz als Paraformaldehyd ist bevorzugt. In den angegebenen Lösungsmitteln liegt Paraformaldehyd z. T. suspendiert und z. T. in depolymerisierter Form gelöst vor. Die Depolymerisation kann beispielsweise durch Zugabe geringer Mengen an Cyanidionen

beschleunigt werden. Der Formaldehyd wird in äquimolaren Mengen oder in einem Überschuß von bis zu 10 Mol-%, bezogen auf die Methylen-Verbindung, eingesetzt. Im bevorzugter Weise wird ein molarer Überschuß von 5 - 10 % an Formaldehyd eingesetzt. Höhere Überschüße an Formaldehyd sind möglich, bringen aber im allgemeinen keine weitere Erhöhung der Ausbeute, während geringere als äquimolare Mengen an Formaldehyd zu einem unvollständigen Umsatz der Methylen-Verbindung führen.

Die Methylen-Verbindung wird erfindungsgemäß zu dem Gemisch der vorgelegten Reaktionsteilnehmer Formaldehyd, Kondensationskatalysator, Hydrierkatalysator und Wasserstoff, das zuvor auf die gewünschte Reaktionstemperatur gebracht wurde, zugefügt. Dieses Zufügen erfolgt im allgemeinen annähernd in dem Maße, wie die Reaktion fortschreitet oder etwas langsamer als der Fortschritt der Reaktion. Der Fortschritt der Reaktion kann hierbei in einfacher Weise am Verbrauch des Wasserstoffs verfolgt werden. Dies geschieht beispielsweise dadurch, daß man den Druck während der Reaktion konstant hält und den zur Konstanthaltung erforderlichen Wasserstoff über einen Durchflußzähler, wie ein Rotameter, bestimmt. Das Zufügen der Methylen-Verbindung kann hierbei in geeigneten kleinen Portionen oder nahezu kontinuierlich, beispielsweise durch eine Dosierpumpe erfolgen. Zur Verfolgung des Fortschritts der Reaktion wird hierbei davon ausgegangen, daß zur Hydrierung der im Reaktionsgemisch entstehenden Kondensationsverbindung aus Formaldehyd und Methylen-Verbindung ein Mol Wasserstoff pro Mol Methylen-Verbindung zur Hydrierung gebraucht wird.

Das erfindungsgemäße Verfahren wird in flüssiger Phase durchgeführt. Als Lösungs- bzw. Verdünnungsmittel seien beispielsweise genannt: Niedere Alkohole, wie Methanol, Ethanol, Propanol, Butanol, Ester organischer Säuren, wie Essigsäureethylester, Nitrile, wie Acetonitril, organische Säuren, wie Essigsäure, Ketone wie Aceton oder Methylethylketon und andere Lösungsmittel, wie beispielsweise Wasser. In bevorzugter Weise werden niedere Alkohole, wie Methanol oder Ethanol, Essigsäureethylester oder Methylethylketon, in besonders bevorzugter Weise Methanol eingesetzt. Während Wasser als Lösungsmittel bei leicht hydrolysierbaren Verbindungen ungünstigere Ergebnisse bringt als die genannten organischen Lösungs- bzw. Verdünnungsmittel, ist es nicht erforderlich, das bei der Kondensationsreaktion entstehende Wasser aus dem Reaktionsgemisch zu entfernen. Andererseits kann bei schwer hydrolysierbaren Verbindungen wie z. B. Barbitursäure, Wasser mit Vorteil als Reaktionsmedium verwendet werden. Die Menge des eingesetzten Lösungs- bzw. Verdünnungsmittels kann in weiten Grenzen variiert werden. Beispielsweise sei eine Menge von 50 - 2000 ml pro Mol Methylen-Verbindung, bevorzugt eine Menge von 150 - 1000 ml, besonders bevorzugt eine Menge von 250 - 500 ml, genannt.

Das Lösungs- bzw. Verdünnungsmittel kann ganz oder teilweise mit den übrigen vorzulegenden Reaktionsteilnehmern vorgelegt werden, wobei im letzteren Falle der Rest zum Lösen oder Verdünnen der einzudosierenden Methylen-Verbindung dient.

Das erfindungsgemäße Verfahren wird beispielsweise so durchgeführt, daß Paraformaldehyd, der Kondensationskatalysator und der Hydrierkatalysator im Lösungs- bzw. Verdünnungsmittel in einem Autoklaven vorgelegt werden. Unter Rühren wird sodann das Reaktionsgemisch auf die gewünschte Reaktionstemperatur gebracht und der gewünschte Wasserstoffpartialdruck aufgepreßt. Dann wird die Methylen-Verbindung in konzentrierter oder verdünnter Form zugepumpt. Die optimale Geschwindigkeit des Zupumpens liegt hierbei möglichst an der Reaktionsgeschwindigkeit, die in der beschriebenen Weise am Fortschritt der Hydrierung beobachtet werden kann und in Vorversuchen auf die gewünschte Temperatur, die gewünschte Verdünnung, den gewünschten Wasserstoffdruck sowie die Art und Menge der Katalysatoren abgestimmt werden kann. Nach dem Einpumpen der gesamten Menge der Methylen-Verbindung wird im allgemeinen noch etwas nachgerührt, wobei gegebenenfalls noch eine geringe nachträgliche Wasserstoffaufnahme beobachtet wird. Nach dem Abkühlen und Entspannen des Autoklaven wird der Hydrierkatalysator abfiltriert und das Filtrat durch Destillation, Kristallisation oder eine andere geeignete Aufarbeitungsmethode aufgetrennt. Für den Fall, daß die Methylen-Verbindung im gewählten Lösungsmittel sehr schwer löslich ist, kann sie bereits zu Beginn gemeinsam mit den anderen Reaktionsteilnehmern vorgelegt werden. Auch in diesem Fall geht die Methylen-Verbindung erfindungsgemäß erst im Verlauf der Reaktion in die flüssige Phase über und wird dort umgesetzt.

**Beispiel 1**

In einem 1,3-Liter-V4A-Autoklaven werden 48,5 g (1,58 mol) gepulverter Paraformaldehyd (98 %-ig), 3,3 g (0,04 mol) Natriumacetat und 8,0 g eines 5 %-igen Pd/C-Katalysators in 300 ml Methanol vorgelegt. Der Autoklav wird mit Stickstoff gespült und unter Rühren auf 80° C aufgeheizt. Es wird ein Wasserstoffdruck von 10 bar eingestellt und im Verlauf von 2 Stunden wird eine Lösung von 143,0 g (1,44 mol) Cyanessigsäuremethylester in 400 ml Methanol eingepumpt. Durch Nachführen von Wasserstoff über ein Reduzierventil wird der Druck von 10 bar aufrecht erhalten. Anschließend wird noch bis zum Ende der Wasserstoffaufnahme gerührt (ca. 3

Stunden). Nach dem Abkühlen auf Raumtemperatur wird der Autoklav entspannt und der Autoklaveninhalt vom Kontakt abfiltriert, der mit Methanol gewaschen, erneut eingesetzt werden kann. Das klare hellgelbe Filtrat wird über Natriumsulfat getrocknet und über eine 30 cm-Vigreux-Kolonne destilliert. Nachem das Methanol bei Normaldruck abdestilliert ist, wird der gesamte Rest bei 16 mbar überdestilliert. Man erhält bei einer Kopftemperatur zwischen 80 und 90° C 153,7 g Destillat, das nach gaschromatischer Analyse 99,5 %-ig an 2-Cyanpropionsäuremethylester ist. Dies entspricht einer Ausbeute von 94,0 % der theoretischen Ausbeute, bezogen auf eingesetzten Cyanessigsäuremethylester. Im Destillationskolben bleibt ein hochviskoser Rückstand von 6 g.

**Beispiel 2**

Es wird wie im Beispiel 1 verfahren. Jedoch werden statt Natriumacetat 3,3 g (0,06 mol) Essigsäure eingesetzt und anstelle des Cyanessigsäuremethylesters 167,2 g (1,44 Mol) Acetessigsäuremethylester in methanolischer Lösung eingepumpt. Nachdem bei der Aufarbeitung des Ansatzes Methanol und Essigsäure abdestilliert sind, werden 189,0 g 95 %-iger 2-Methylacetessigsäuremethylester erhalten. Dies entspricht einer Ausbeute von 95,9 % der theoretischen Ausbeute, bezogen auf eingesetzten Acetessigsäuremethylester.

Das Destillat enthält 2,1 % 2-Methoximethylacetessigsäuremethylester als Nebenprodukt. Im Destillationskolben bleibt ein Rückstand von 3,0 g.

**Beispiel 3**

Es wird wie im Beispiel 2 verfahren, statt Acetessigsäuremethylester werden jedoch 230,4 g (1,44 mol) Malonsäurediethylester eingepumpt. Nach analoger Aufarbeitung werden bei der Destillation 230,0 g 90 %-iger Methylmalonsäurediethylester erhalten. Dies entspricht einer Ausbeute von 82,6 % der theoretischen Ausbeute, bezogen auf eingesetzten Malonsäurediethylester. Das Destillat enthält als Nebenprodukt noch 9,3 % Methoximethylmalonsäurediethylester. Im Destillationskolben bleibt ein Rückstand von 3,1 g.

**Beispiel 4**

Es wird wie im Beispiel 1 verfahren, aber statt Cyanessigsäuremethylester werden 144,0 g (1,44 mol) Acetylaceton eingepumpt. Bei der Destillation werden 147,4 g 98 %-iges 3-Methylpentandion-2,4 erhalten. Dies entspricht einer Ausbeute von 88,0 % der theoretischen Ausbeute, bezogen auf eingesetztes Acetylaceton. Das Destillat enthält 1,3 % 3-Methoximethylpentandion-2,4. Im Destillationskolben bleibt ein Rückstand von 10,3 g.

**Beispiel 5**

In einem 1,3-Liter-V4A-Autoklaven werden 11,3 g (0,37 mol) gepulverter Paraformaldehyd (98 %-ig), 0,8 g (0,01 mol) Natriumacetat und 3,7 g eines 5 %-igen Pd/C-Katalysators in 200 ml Methyl-ethyl-keton vorgelegt. Der Autoklav wird mit Stickstoff gespült und unter Rühren auf 80° C aufgeheizt. Es wird ein Wasserstoffdruck von 10 bar eingestellt und im Verlauf von 2 Stunden eine Lösung von 54 g (0,33 mol) 4-Nitrobenzylcyanid im 370 ml Methyl-ethyl-keton eingepumpt.

Temperatur und Wasserstoffdruck werden bis zur Beendigung der Wasserstoffaufnahme beibehalten (ca. 1,5 Std.). Bei der Destillation werden bei 150 - 160° C/1,3 mbar 29 g eines Produktes erhalten, das zu 97 % aus 2-(4-Nitrophenyl)-propionitril und 3 % Ausgangsprodukt besteht. Es kann aus Methanol umkristallisiert werden und hat einen Schmelzpunkt von 74 - 75° C. Ausbeute 48,4 %.

**Beispiel 6**

Es wird wie im Beispiel 1 verfahren, statt Acetessigsäuremethylester werden 95,0 g (1,44 mol) Malodinitril in methanolischer Lösung eingepumpt. Nach analoger Aufarbeitung werden bei der Destillation 95,1 g 93 %-iges Methylmalodinitril erhalten. Als Nebenprodukt wird das Methoximethylmalodinitril gebildet. Der Destillationsrückstand beträgt 6,5 g. Die Ausbeute beträgt 77 % der theoretischen Ausbeute.

**Beispiel 7**

In einem 1,3 l-V4A-Autoklaven werden 184 g (1,44 mol) Barbitursäure, 48,5 g (1,58 mol) gepulverter Paraformaldehyd (98 %-ig), 3,3 g (0,04 mol) Natriumacetat, und 16 g eines 5 %-igen Pd/C-Katalysators in 700 ml Wasser aufgeschlämmt. Der Autoklav wird mit Stickstoff gespült und unter Rühren auf 80° C aufgeheizt. Es wird ein Wasserstoffdruck von 10 bar eingestellt und bis zur Druckkonstanz gerührt.

Dem ausgenommenen Autoklaveninhalt wird 1 l Wasser zugesetzt. Es wird zum Rückfluß erhitzt und heiß vom Ungelösten abfiltriert. Aus dem Filtrat kristallisieren 130,7 g 5-Methylbarbitursäure aus (Fp: 205 - 217° C). Beim Einengen der Mutterlauge werden weitere 75,9 g (Fp: 195 - 202° C) gewonnen.

**Vergleichsbeispiele**

Führt man die in den Beispielen 1 - 5 beschriebenen Versuche in der Weise durch, daß man die Methylen-Verbindung nicht zupumpt, sondern mit den übrigen Reaktionskomponenten vorlegt und im übrigen wie in den Beispielen geschildert verfährt, werden folgende Ergebnisse erhalten:

**Vergleich zu Beispiel 1**

Nachdem alles Methanol abdestilliert ist, erhält man 175,7 g eines zähflüssigen Rückstandes, der bei 16 mbar bis zu einer Sumpftemperatur von 200° C nicht destillierbar ist. Im abdestillierten Methanol sind nur Spuren von Cyanessigsäuremethylester und 2-Cyanpropionsäuremethylester nachweisbar.

**Vergleich zu Beispiel 2**

Nachdem alles Methanol abdestilliert ist, werden 90,5 g Destillat erhalten, das 84,3 % 2-Methylacetessigsäuremethylester enthält. Das entspricht einer Ausbeute von 45,7 % der theoretischen Ausbeute, bezogen auf eingesetzten Acetessigsäuremethylester. Das Destillat enthält neben etwas Acetessigsäuremethylester 9,1 % 2-Methoximethylacetessigsäuremethylester. Es bleibt ein nicht destillierbarer Rückstand von 77,5 g.

**Vergleich zu Beispiel 3**

Nachdem der Alkohol abdestilliert ist, werden 229,1 g Destillat erhalten, das 10,5 Gew.-% Methylmalonsäurediethylester, 82,7 Gew.-% Malonsäurediethylester und 3,3 Gew.-% Methoximalonsäurediethylester enthält. Dies entspricht einer Ausbeute von 9,6 % der theoretischen Ausbeute, bezogen auf eingesetzten Malonsäurediethylester. Es bleibt im Destillationskolben ein Rückstand von 1,5 g.

**Vergleich zu Beispiel 4**

Nachdem alles Methanol abdestilliert ist, werden 69,5 g Destillat erhalten, das 88,3 Gew.-% 3-Methylpentandion-2,4 sowie verschiedene höher siedende Komponenten enthält. Dies entspricht einer Ausbeute von 37,4 % der theoretischen Ausbeute, bezogen auf eingesetztes Acetylaceton. Es bleibt ein Destillationsrückstand von 81,4 g.

**Vergleich zu Beispiel 5**

Nachdem das Lösungsmittel abdestilliert ist, bleiben 57,4 g eines Rückstandes, der nach NMR-Spektroskopie und dünnschichtchromatographischer Analyse nur Spuren 2-(4-Nitrophenyl)-propionitril enthält.

**Vergleich zu Beispiel 6**

Nachdem alles Methanol abdestilliert ist, erhält man 102 g eines dunkelbraunen, zähflüssigen, stark nach Amin riechenden Rückstands, der nicht destillierbar ist.

**Patentansprüche**

1. Verfahren zur Herstellung von monomethylsubstituierten Methylen-Verbindungen der Formel

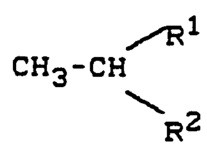

durch Methylierung von Methylen-Verbindungen der Formel

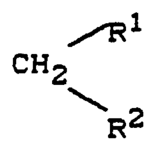

in der

$R^1$ und $R^2$ unabhängig voneinander für -CN, -CO-$R^3$, -$SO_2$-$R^3$ und -$NO_2$ stehen und $R^1$ zusätzlich -Aryl-$(R^1)_n$ bedeuten kann, wobei

$R^3$ -OH, Alkyl, Aralkyl, Aryl, Alkoxy, Aralkoxy, Aryloxy oder gegebenenfalls durch Alkyl und/oder Aralkyl und/oder Aryl substituiertes Amino bedeuten und weiterhin zwei Reste $R^3$ gemeinsam eine Alkylengruppe, der Rest eines aliphatischen Diols oder eines aliphatischen Diamins oder die Gruppe -NH-CO-NH- sein können und

n für 1, 2 oder 3 steht,

durch Reaktion mit Formaldehyd und Wasserstoff in Gegenwart eines Kondensationskatalysators und eines

Hydrierkatalysators bei erhöhter Temperatur, dadurch gekennzeichnet, daß die Reaktion in einem Lösungsmittel durchgeführt wird und die Methylen-Verbindung im Verlauf der Reaktion der flüssigen Phase des Gemisches der Reaktionsteilnehmer zugefügt wird oder eine im Lösungsmittel sehr schwer lösliche Methylen-Verbindung gemeinsam mit den anderen Reaktionsteilnehmern vorgelegt wird, die im Verlauf der Reaktion in die flüssige Phase übergeht.

2. Verfahren nach Anspruch 1, dadurch gekennzeichnet, daß die Reaktion in Gegenwart von Methanol, Ethanol, Essigsäureethylester oder Methylethylketon als Lösungsmittel durchgeführt wird.

3. Verfahren nach Anspruch 1 und 2, dadurch gekennzeichnet, daß die Reaktion in Gegenwart von Methanol als Lösungsmittel durchgeführt wird.

4. Verfahren nach Anspruch 1, dadurch gekennzeichnet, daß der Formaldehyd in Form von Paraformaldehyd eingesetzt wird.

5. Verfahren nach Anspruch 1 - 4, dadurch gekennzeichnet, daß als Methylen-Verbindung solche der Formel

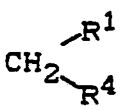

eingesetzt werden, in der

$R^1$ die in Anspruch 1 genannte Bedeutung hat und

$R^4$ für -CN oder $-CO-R^3$ steht, wobei $R^3$ die in Anspruch 1 genannte Bedeutung hat.

6. Verfahren nach Anspruch 1 - 4, dadurch gekennzeichnet, daß als Methylen-Verbindung solche der Formel

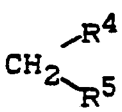

eingesetzt werden, in der

$R^4$ und $R^5$ unabhängig voneinander für -CN oder $-CO-R^3$ stehen, wobei $R^3$ die in Anspruch 1 genannte Bedeutung hat.

7. Verfahren nach Anspruch 1 - 4, dadurch gekennzeichnet, daß als Methylen-Verbindung solche der Formel

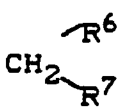

eingesetzt werden, in der

$R^6$ und $R^7$ unabhängig voneinander -CN, $-CO-C_1-C_4$-Alkyl oder $CO-O-C_1-C_4$-Alkyl bedeuten.

**Claims**

1. Process for the preparation of monomethyl-substituted methylene compounds of the formula

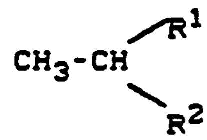

by methylation of methylene compounds of the formula

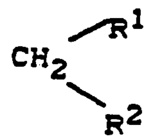

in which

$R^1$ and $R^2$ independently of one another represent -CN, $-CO-R^3$, $-SO_2-R^3$ and $-NO_2$ and

$R^1$ can additionally denote -aryl-$(R^1)_n$, wherein

$R^3$ denotes -OH, alkyl, aralkyl, aryl, alkoxy, aralkoxy or aryloxy, or amino which is optionally substituted by alkyl and/or aralkyl and/or aryl, and furthermore

two radicals $R^3$ together can be an alkylene group, the radical of an aliphatic diol or of an aliphatic diamine or the group -NH-CO-NH- and n represents 1, 2 or 3,

by reaction with formaldehyde and hydrogen in the presence of a condensation catalyst and a hydrogenation catalyst at elevated temperature, characterised in that the reaction is carried out in a solvent and the methylene compound is introduced into the liquid phase of the mixture of reactants in the course of the reaction, or a methylene compound which is very sparingly soluble in the solvent is taken at the start together with the other reactants, and passes into the liquid phase in the course of the reaction.

2. Process according to Claim 1, characterised in that the reaction is carried out in the presence of methanol, ethanol, ethyl acetate or methyl ethyl ketone, as the solvent.

3. Process according to Claims 1 and 2, characterised in that the reaction is carried out in the presence of methanol, as the solvent.

4. Process according to Claim 1, characterised in that the formaldehyde is used in the form of paraformaldehyde.

5. Process according to Claims 1 - 4, characterised in that methylene compounds of the formula

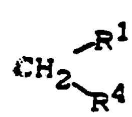

in which

$R^1$ has the meaning given in Claim 1 and

$R^4$ represents -CN or -CO-$R^3$,

wherein

$R^3$ has the meaning given in Claim 1, are used.

6. Process according to Claims 1 - 4, characterised in that methylene compounds of the formula

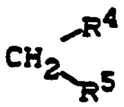

in which

$R^4$ and $R^5$ independently of one another represent -CN or -CO-$R^3$,

wherein

$R^3$ has the meaning given in Claim 1, are used.

7. Process according to Claims 1 - 4, characterised in that methylene compounds of the formula

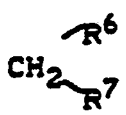

in which

$R^6$ and $R^7$ independently of one another denote -CN, -CO-$C_1$-$C_4$-alkyl or CO-O-$C_1$-$C_4$-alkyl, are used.

**Revendications**

1. Procédé de production de composés méthyléniques à substituant monométhyle, de formule

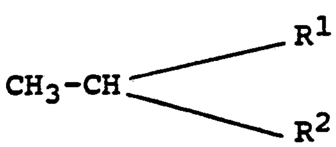

par méthylation de composés méthyléniques de formule

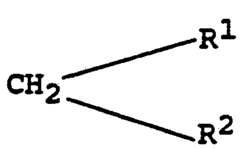

formules dans lesquelles

$R^1$ et $R^2$ représentent, indépendamment l'un de l'autre, -CN, -CO-$R^3$, -$SO_2$-$R^3$ et -$NO_2$ et $R^1$ peut désigner en outre un groupe aryle $(R^1)_n$, où

$R^3$ représente un groupe -OH, alkyle, aralkyle, aryle, alkoxy, aralkoxy, aryloxy ou un groupe amino éventuellement substitué par un radical alkyle et/ou aralkyle et/ou aryle et, en outre, deux restes $R^3$ peuvent former conjointement un groupe alkylène, le reste d'un diol aliphatique ou d'une diamine aliphatique ou le groupe -NH-CO-NH et

$\underline{n}$ à la valeur 1, 2 ou 3,

par réaction avec le formaldéhyde et l'hydrogène en présence d'un catalyseur de condensation et d'un catalyseur d'hydrogénation, à température élevée, qui est caractérisé en ce que la réaction est conduite dans un solvant et le composé méthylénique est ajouté au cours de la réaction à la phase liquide du mélange de partenaires réactionnels, ou bien on introduit au préalable conjointement avec les autres partenaires réactionnels un composé méthylénique, très difficilement soluble dans le solvant, qui passe dans la phase liquide au cours de la réaction.

2. Procédé suivant la revendication 1, caractérisé en ce qu'on conduit la réaction en présence de méthanol, d'éthanol, d'ester éthylique d'acide acétique ou de méthyléthylcétone comme solvant.

3. Procédé suivant les revendications 1 et 2, caractérisé en ce qu'on conduit la réaction en présence de méthanol comme solvant.

4. Procédé suivant la revendication 1, caractérisé en ce qu'on utilise le formaldéhyde sous la forme de paraformaldéhyde.

5. Procédé suivant les revendications 1 à 4, caractérisé en ce qu'on utilise comme composé méthylénique un composé de formule

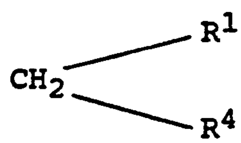

dans laquelle

$R^1$ a la définition indiquée dans la revendication 1 et

$R^4$ représente un groupe -CN ou un groupe -CO-$R^3$, dans lequel $R^3$ a la définition mentionnée dans la revendication 1.

6. Procédé suivant les revendications 1 à 4, caractérisé en ce qu'on utilise comme composé méthylénique des composés de formule

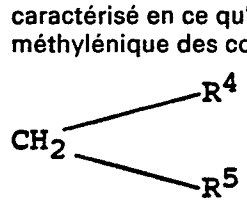

dans laquelle

$R^4$ et $R^5$ représentent, indépendamment l'un de l'autre, un groupe -CN ou -CO-$R^3$ où $R^3$ a la définition indiquée dans la revendication 1.

7. Procédé suivant les revendications 1 à 4, caractérisé en ce qu'on utilise comme composé méthylénique des composés de formule

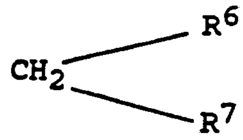

dans laquelle

$R^6$ et $R^7$ représentent, indépendamment l'un de l'autre, un groupe -CN, -CO-(alkyle en $C_1$ à $C_4$) ou -CO-O-(alkyle en $C_1$ à $C_4$).